Europäisches Patentamt

European Patent Office

Office européen des brevets

(19)

(11)  EP 0 698 614 A1

(12)  EUROPEAN PATENT APPLICATION

(43) Date of publication:
28.02.1996  Bulletin 1996/09

(51) Int. Cl.⁶: **C07K 7/08**

(21) Application number: 95111533.6

(22) Date of filing: 21.07.1995

(84) Designated Contracting States:
AT BE CH DE DK ES FR GB GR IE IT LI LU MC NL
PT SE

(30) Priority: 25.07.1994 JP 172699/94

(71) Applicant: KYOWA HAKKO KOGYO CO., LTD.
Chiyoda-ku,  Tokyo 100 (JP)

(72) Inventors:
• Yamasaki, Motoo
Machida-shi,  Tokyo 194 (JP)

• Shibata, Kenji
Kawasaki-shi,  Kanagawa 215 (JP)
• Tanaka, Takeo
Machida-shi,  Tokyo 194 (JP)
• Matsuda, Yuzuru
Koganei-shi,  Tokyo 184 (JP)

(74) Representative: VOSSIUS & PARTNER
D-81675 München (DE)

(54)  **Endothelin-antagonizing peptide**

(57)  Disclosed is a peptide compound represented by the following formula (I):

$$\text{Gly--Asn--Trp--His--Gly--Thr--Ala--Pro--Asp--Trp--} \atop \text{Phe--Phe--Asn--Tyr--Tyr--Trp--X} \qquad \text{(I)}$$

wherein X represents methoxy, benzyloxy, or amino, and a pharmaceutically acceptable salt thereof.

EP 0 698 614 A1

## Description

The present invention relates to a peptide which has endothelin-antagonizing activity. The peptide of the present invention has excellent endothelin-antagonizing activity, and is therefore useful for the treatment of hypertension, asthma, cerebral apoplexy, angina pectoris, acute renal failure, cardiac infarction, cerebral vasospasm, etc.

Endothelin is a cyclic peptide which possesses a strong, long-lasting vasoconstricting effect, and is thought to be one of the substances responsible for hypertension, asthma, cerebral apoplexy, angina pectoris, acute renal failure, cardiac infarction, and cerebral vasospasm. Consequently, a substance which antagonizes endothelin and inhibits its effects is expected to be useful for the treatment and prevention of these diseases.

It is known that the peptide represented by the following formula (A):

$$\overline{\underset{\displaystyle \text{Phe–Phe–Asn–Tyr–Tyr–Trp–Z}}{\text{Gly–Asn–Trp–His–Gly–Thr–Ala–Pro–Asp–Trp–}}} \qquad \text{(A)}$$

wherein Z represents hydroxy, lower alkoxy, benzyloxy, etc., exhibits endothelin antagonism (WO93/13218). However, only lower alkoxy and benzyloxy are suggested as the substituent Z by the general formula in the above publication, and there is no disclosure about a concrete peptide having methoxy or benzyloxy moiety. In addition, a compound wherein Z is amino is not known.

According to the present invention, there is provided a peptide compound represented by the following formula (I):

$$\overline{\underset{\displaystyle \text{Phe–Phe–Asn–Tyr–Tyr–Trp–X}}{\text{Gly–Asn–Trp–His–Gly–Thr–Ala–Pro–Asp–Trp–}}} \qquad \text{(I)}$$

wherein X represents methoxy, benzyloxy, or amino, or a pharmaceutically acceptable salt thereof.

The peptide compound represented by the above formula (I) is hereinafter referred to as Compound (I), and the same applies to the compounds of other formula numbers.

The pharmaceutically acceptable salts of Compound (I) include pharmaceutically acceptable acid addition salts, metal salts, and organic base addition salts.

Examples of the pharmaceutically acceptable acid addition salts are inorganic acid addition salts such as hydrochloride, sulfate, and phosphate, and organic acid addition salts such as acetate, maleate, fumarate, tartrate, and citrate. Examples of the pharmaceutically acceptable metal salts are alkali metal salts such as the sodium salt and potassium salt, alkaline earth metal salts such as the magnesium salt and calcium salt, aluminum salts, and zinc salts. Examples of the pharmaceutically acceptable organic base addition salts are salts with primary amines such as methylamine, ethylamine, and aniline, secondary amines such as dimethylamine, diethylamine, pyrrolidine, piperidine, morpholine, and piperazine, and tertiary amines such as trimethylamine, triethylamine, N,N-dimethylaniline, and pyridine, and ammonium salts.

The present invention is described in detail below.

The abbreviations for the amino acids and their protective groups used in this specification follow the recommendations of the IUPAC-IUB Joint Commission relating to biochemical nomenclature [Eur. J. Biochem., 138, 9 (1984)].

Unless otherwise provided, the following abbreviations indicate the corresponding amino acids and protective groups.

Gly:       glycine
Ala:       L-alanine
Thr:       L-threonine
Pro:       L-proline
Asp:       L-aspartic acid
Asn:       L-asparagine
Asx:       L-aspartic acid or L-asparagine
His:       L-histidine
Phe:       L-phenylalanine

Tyr:    L-tyrosine
Trp:    L-tryptophan
OBzl:    benzyloxy
OMe:    methoxy

The following abbreviation indicates the corresponding side-chain-protected amino acids.

H-Trp-OMe:    L-tryptophan methyl ester
H-Trp-OBzl:    L-tryptophan benzyl ester
H-Trp-NH$_2$:    L-tryptophan amide

The following abbreviations indicate the corresponding reaction solvents and reagents.

PyBOP:    benzotriazol-1-yloxytripyrrolidinophosphonium-hexafluorophosphate
HOBt:    N-hydroxybenzotriazole
NMM:    N-methylmorpholine
DMF:    N,N-dimethylformamide
TFA:    trifluoroacetic acid

The processes for producing Compounds (I) of the present invention are described below.
Compound (I) can be prepared according to the following reaction scheme.

Gly–Asn–Trp–His–Gly–Thr–Ala–Pro–Asp–Trp–
Phe–Phe–Asn–Tyr–Tyr–Trp–OH

(RES–701–1)

↓

Gly–Asn–Trp–His–Gly–Thr–Ala–Pro–Asp–Trp–
Phe–Phe–Asn–Tyr–Tyr–OH    (B)

↓ H–Trp–X (II)

Gly–Asn–Trp–His–Gly–Thr–Ala–Pro–Asp–Trp–
Phe–Phe–Asn–Tyr–Tyr–Trp–X    (I)

(In the formulae, X has the same meaning as defined above.)

Compound (B) can be obtained by treating RES-701-1 (WO93/13218) with a protease such as carboxypeptidase.

Then, Compound (I) can be obtained by condensing Compound (B) with Compound (II) using a condensing agent (for example, PyBOP/HOBt/NMM).

The thus obtained Compound (I) may be purified by high pressure liquid chromatography (referred to as HPLC hereinafter) using a C-4, C-8, or C-18 reverse phase silica gel column, column chromatography such as partition, adsorption resin, silica gel, chemically modified silica gel, reverse phase silica gel, alumina, diatomaceous earth, magnesium silicate, ion-exchange resin, and gel filtration, or thin layer chromatography.

A conventional method is used to obtain a pharmaceutically acceptable salt of Compound (I). That is, an acid addition salt or an organic base addition salt of Compound (I) can be obtained by dissolving Compound (I) in an aqueous solution of a corresponding acid or organic base, and freeze-drying the resultant solution. In addition, a metal salt of Compound (I) can be obtained by dissolving Compound (I) in an aqueous solution containing a corresponding metal ion, and purifying the solution by gel filtration or HPLC.

Examples of Compound (I) are shown in Table 1.

## Table 1

| Compd. No. | Structure |
|---|---|
| (I-1) | ⌐Gly–Asn–Trp–His–Gly–Thr–Ala–Pro–Asp–Trp–<br>                              Phe–Phe–Asn–Tyr–Tyr–Trp–OMe |
| (I-2) | ⌐Gly–Asn–Trp–His–Gly–Thr–Ala–Pro–Asp–Trp–<br>                              Phe–Phe–Asn–Tyr–Tyr–Trp–OBzl |
| (I-3) | ⌐Gly–Asn–Trp–His–Gly–Thr–Ala–Pro–Asp–Trp–<br>                              Phe–Phe–Asn–Tyr–Tyr–Trp–NH$_2$ |

The pharmacological activities of Compound (I) are described below by test examples.

Test Example 1: Endothelin Receptor-Antagonizing Activity

Bovine cerebellum tissue was homogenized at 4°C by using POLYTRON (type PT10/35, manufactured by Kinematica Gmbh Co.) in a buffer solution A (1mM NaHCO$_3$, 5 mM ethylenediaminetetraacetic acid, 5 µg/ml leupeptin, 5 µg/ml pepstatin A, 40 µM phenylmethylsulfonyl fluoride, pH 8.3).

The obtained suspension was centrifuged for 10 minutes at 8,000 G and 4°C, and the resulting supernatant was centrifuged for 60 minutes at 40,000 G and 4°C to give pellets. The obtained pellets were suspended in a buffer solution A and again centrifuged for 60 minutes at 40,000 G and 4°C. The resulting solid substance was prepared as a suspension containing 2 mg/ml of protein, and the suspension was used as a membrane fraction liquid. A membrane fraction solution was prepared by adding 7 µl of the membrane fraction liquid per 1 ml of a buffer solution B (50 mM Tris-HCl, 1 mM ethylenediaminetetraacetic acid, 0.2% bovine serum albumin, pH 7.6). $^{125}$I-Endothelin-1 (approx. 30,000 cpm) was added to the membrane fraction solution containing unlabelled endothelin-1 (final concentration 100 nM) or any one of the test compounds, or containing neither of them. These mixtures were incubated at 25°C for 2 hours, and then filtered with a GF/B glass filter (produced by Whatman Co.). After washing the filter with a buffer solution C (50 mM Tris-HCl, 1 mM ethylenediaminetetraacetic acid, pH 7.6), the radioactivity on the glass filter was measured to determine the amount of the receptor and the non-specific bound $^{125}$I-endothelin. The inhibition rate against endothelin receptor binding was calculated according to the following equation:

$$\text{Inhibition rate (\%)} = (C - A/C - B) \times 100$$

A:    Radioactivity in the presence of one of the test compounds
B:    Radioactivity in the presence of unlabelled endothelin-1
C:    Radioactivity in the absence of both of the test compound and unlabelled endothelin-1

The results are shown in Table 2.

Table 2

| Compound No. | $IC_{50}$ (nM) |
|---|---|
| (I-1) | 15 |
| (I-2) | 140 |
| (I-3) | 49 |
| RES-701-1 | 10 |
| $IC_{50}$: Concentration causing 50 % inhibition of endothelin-1 binding | |

Test Example 2: Endothelin Receptor-Antagonizing Activity

The inhibition rate against endothelin receptor binding was calculated by the same method as in Test Example 1, except for using bovine lung tissue instead of the bovine cerebellum tissue used in Test Example 1, and adding RES-701-1 to the membrane fraction solution at a concentration of 1.5 μg/ml.

Table 3

| Compound No. | $IC_{50}$ (nM) |
|---|---|
| (I-1) | 58 |
| (I-2) | >470 |
| (I-3) | >490 |
| RES-701-1 | >1000 |
| $IC_{50}$: Concentration causing 50 % inhibition of endothelin-1 binding | |

Examples and Reference Example of the present invention are described below.

Example 1: Synthesis of Compound (I-1)

0.1 ml of a DMF solution containing 0.84 mg of PyBOP, 0.1 ml of a DMF solution containing 0.22 mg of HOBt, and 0.1 ml of a DMF solution containing 0.3 μl of NMM were added to 0.5 ml of a DMF solution containing 1 mg of Compound (B) obtained in Reference Example 1, followed by standing at 0°C for 10 minutes. Then, 0.1 ml of a DMF solution containing 0.69 mg of H-Trp-OMe·HCl and 0.3 μl of NMM was added thereto, and the mixture was stirred for 6 hours at room temperature. 0.1 ml of 2M acetic acid was added thereto, and the crude product was subjected to HPLC using a reverse phase column (CAPCELL PACK C18, 250 mm x 30 mm I.D., manufactured by Shiseido). The elution was carried out with a linear concentration gradient pattern using a 0 to 90% aqueous acetonitrile solution containing 0.1% TFA, and upon detection at 220 nm, to give fractions containing the entitled Compound (I-1). These fractions were lyophilized to give 30 μg of Compound (I-1).

MS analysis [FABMS]: 2056 (M + H)
Amino acid analysis: Found (Theoretical) Gly 2.1 (2), Ala 1.0 (1), Asx 2.1 (3), His 1.0 (1), Pro 1.0 (1), Thr 1.0 (1), Tyr 1.8 (2), Phe 1.8 (2), Trp not analyzed

Example 2: Synthesis of Compound (I-2)

10 μl of a DMF solution containing 84 μg of PyBOP, 10 μl of a DMF solution containing 22 μg of HOBt, and 10 μl of a DMF solution containing 0.03 μl of NMM were added to 55 μl of a DMF solution containing 100 μg of Compound (B) obtained in Reference Example 1, followed by standing at 0°C for 10 minutes. Then, 10 μl of a DMF solution containing 89 μg of H-Trp-OBzl·HCl and 0.03 μl of NMM was added thereto, and the mixture was stirred for 54 hours at 4°C. 10 μl of 2M acetic acid was added thereto, and the crude product was purified in the manner similar to that in Example 1 to give 50 μg of Compound (I-2).

MS analysis [FABMS]: 2132 (M + H)

Amino acid analysis: Found (Theoretical) Gly 2.2 (2), Ala 1.0 (1), Asx 2.2 (3), His 1.1 (1), Pro 1.2 (1), Thr 1.0 (1), Tyr 1.9 (2), Phe 1.9 (2), Trp not analyzed

Example 3: Synthesis of Compound (I-3)

10 μl of a DMF solution containing 84 μg of PyBOP, 10 μl of a DMF solution containing 22 μg of HOBt, and 10 μl of a DMF solution containing 0.03 μl of NMM were added to 55 μl of a DMF solution containing 100 μg of Compound (B) obtained in Reference Example 1, followed by standing at 0°C for 10 minutes. Then, 10 μl of a DMF solution containing 32 μg of H-Trp-NH$_2$·HCl and 0.03 μl of NMM was added thereto, and the mixture was stirred for 54 hours at 4°C. 10 μl of 2M acetic acid was added thereto, and the crude product was purified in the manner similar to that in Example 1 to give 62 μg of Compound (I-3).

MS analysis [FABMS]: 2041 (M + H)

Amino acid analysis: Found (Theoretical) Gly 2.2 (2), Ala 1.0 (1), Asx 2.1 (3), His 1.0 (1), Pro 1.1 (1), Thr 1.0 (1), Tyr 1.9 (2), Phe 1.9 (2), Trp not analyzed

Reference Example 1: Synthesis of Compound (B)

7.1 mg of RES-701-1 was dissolved in 2.84 ml of methanol, and 25.56 ml of a 0.1M Tris-HCl buffer solution (pH 8.0) and then about 0.3 mg of carboxypeptidase A (C-9762, produced by Sigma Co.) were added thereto, followed by stirring at 37°C for one night. The reaction mixture was acidified by addition of an appropriate amount of hydrochloric acid, and the crude product was subjected to reverse phase HPLC equipped with a NUCLEOSIL 5C18 (250 x 20 mm I.D., manufactured by Chemco Inc.) with a linear concentration gradient pattern wherein an increase in the concentration of acetonitrile in the 0.1% TFA solution from 0% to 50% was effected in 30 minutes, at a flow rate of 10 ml/min, to give 3.5 mg of Compound (B).

MS analysis [FABMS]: 1858 (M + H)

Amino acid analysis: Found (Theoretical) Gly 2.2 (2), Ala 1.0 (1), Asx 2.6 (3), His 0.9 (1), Pro 1.1 (1), Thr 1.0 (1), Tyr 1.8 (2), Phe 1.9 (2), Trp not analyzed

**Claims**

1. A peptide compound represented by the following formula (I):

$$\text{Gly--Asn--Trp--His--Gly--Thr--Ala--Pro--Asp--Trp--}$$
$$\text{Phe--Phe--Asn--Tyr--Tyr--Trp--X} \quad \text{(I)}$$

wherein X represents methoxy, benzyloxy, or amino, or a pharmaceutically acceptable salt thereof.

EP 0 698 614 A1

European Patent Office

EUROPEAN SEARCH REPORT

Application Number

## DOCUMENTS CONSIDERED TO BE RELEVANT

EP 95111533.6

| Category | Citation of document with indication, where appropriate, of relevant passages | Relevant to claim | CLASSIFICATION OF THE APPLICATION (Int. Cl. 6) |
|---|---|---|---|
| X,D | WO - A - 93/13 218 (KYOWA HAKKO KOGYO CO., LTD.) * Claim 2 * | 1 | C 07 K 7/08 |

TECHNICAL FIELDS SEARCHED (Int. Cl.6)

C 07 K
A 61 K

The present search report has been drawn up for all claims

| Place of search | Date of completion of the search | Examiner |
|---|---|---|
| VIENNA | 24-11-1995 | BÖHM |